# EUROPÄISCHE PATENTANMELDUNG

(11) **EP 1 870 132 A1**
(43) Veröffentlichungstag der Anmeldung: **26.12.2007**
(21) Anmeldenummer: 06013074.7
(22) Anmeldetag: 24.06.2006
(51) Int. Cl.: A61N 5/06

(54) **Apparat zur Bestrahlung eines menschlichen Körpers mit natürlichem Sonnenlicht**

(71) Anmelder: Gödl, Albin, 4400 Steyr-Garsten (AT)
(72) Erfinder: Gödl, Albin, 4400 Steyr-Garsten (AT); Gödl, Johann, 4400 Steyr-Garsten (AT)

(57) **Zusammenfassung**

Es wird ein Bestrahlungsapparat (1) zur Teil- oder Ganzkörperbestrahlung des menschlichen Körpers beschrieben, der die Bestrahlung für kosmetische als auch therapeutische Zwecke mittels einer das ganzheitliche möglichst verzerrungsfreie Frequenzspektrum des natürlichen Sonnenlichts durchläßigen Abdeckung (2) ermöglicht. Dabei wird der Apparat mit seinen wärmegedämmten Umschließungsflächen (4) derart ausgeführt, dass innenliegende verstellbare Flächen (9), die wahlweise als Absorber oder Reflektoren arbeiten, und weitere erfindungsgemäße Konstruktionen die Innentemperaturanpassung bei kühlen als auch heißen Außentemperaturen ermöglichen.

## Beschreibung

Die Erfindung betrifft einen Apparat zur Bestrahlung des menschlichen Körpers mit natürlichem Sonnenlicht.

Sonnenlicht ist für die Gesundheit des menschlichen Körpers nach heutigem Wissensstand unerläßlich, denn viele biologische Prozesse werden nur durch dieses ausgelöst. Der menschliche Körper und die Evolution der Körperzellen entwickelten sich über hunderttausende Jahre hinweg in Wechselwirkung mit dem ganzheitlichen Spektrum des Sonnenlichtes. Unsere Vorfahren hielten sich wesentlich mehr in der freien Natur auf und nahmen daher wesentlich höhere Sonnenlichtdosen auf als wir dies heute tun. Der gesamte menschliche Organismus und seine biologischen Prozesse haben sich an dieses natürliche Strahlungsspektrum angepaßt.

Vor etwa 200 Jahren, mit dem Beginn des industriellen Zeitalters, stellten sich die Arbeits- und Lebensgewohnheiten in zahlreichen Ländern drastisch um. Viele Menschen halten sich seit dem von morgens bis abends auch bei der Arbeit in Gebäuden auf und sind damit dem Sonnenlicht fern. Weitere Gründe für geringere Sonnenlichtdosen sind, daß in den wirtschaftlich höher entwickelten Staaten, meist aus gesellschaftlichen Gepflogenheiten und auch durch die dort herrschenden kühlen klimatischen Verhältnisse, die Haut großteils mit lichtundurchlässiger Kleidung vor dem lebensnotwendigen Sonnenlicht verborgen ist. Dadurch leiden viele Menschen ohne es zu wissen, an chronischem Lichtmangel, der in Wechselwirkung mit falscher Ernährung, sowie psychischem Streß das Energieniveau in den Körperzellen derart schwächt, daß sich daraus ein Symptom entwickelt, welches sich schließlich auf der Organebene manifestiert. Symptome sind jedermann unter dem Namen Krankheit bekannt. Dieser Lichtmangel hat auch dazu geführt, daß Apparate wie Solarien entwickelt wurden. In erster Linie, zwar für kosmetische Zwecke, um einerseits der Haut durch Bräunung gesundes natürliches Aussehen zu geben, und anderseits zum Einsatz bei der Bestrahlung von Hautkrankheiten. Teilerfolge bei diesen Symptombehandlungen sind gegeben, ursächlich sind diese jedoch nicht. Nachteilig wirkt sich dabei aus, daß künstlich erzeugte Lichtspektren niemals dem natürlichen Sonnenlicht gleichen können, da hierbei gewisse Frequenzen fehlen oder Verzerrungen gewisser Frequenzen erfolgen, also kein ganzheitliches Lichtspektrum gegeben ist. Sollte es jemals gelingen Lampen herzustellen, die vergleichbar mit natürlichem Sonnenlicht sind, dann stellt sich auch noch die Frage, wie lange hält dieser Effekt an bis sich wieder Verzerrungen durch Materialermüdung ergeben und dem Körper, der ja ein lebendiger Organismus ist, mit anorganischem, verzerrtem Licht, Schaden zufügt. Künstliches Licht wird daher niemals die Qualität des Sonnenlichtes erreichen.

In PCT/US95/14709, US 5.047.007, und US5.266.070 werden Lichttherapien beschrieben die ebenfalls mit künstlichem Licht arbeiten. Dabei wird ebenfalls die natürliche Ganzheitlichkeit des Lichts verlassen. Niemand weiß, wie sich diese künstlichen Teil-Lichtspektren auf den gesamten Organismus auf Dauer auswirken. Einerseits bewirken Teil-Lichtspektren in gewissen Gesundsheitsbereichen sehrwohl etwas, da ja etwas Licht schon besser zu sein scheint, als gar kein Licht, andererseits sind die durch elektrische Lampen und Kabel entstehenden Magnetfelder - auch als Elektrosmog bekannt - unnatürlichen Ursprungs, und beeinflussen die natürlichen Zellfunktionen des Körpers während der Therapie negativ. DE 3535777 A1 beschreibt eine Vorrichtung zur Bestrahlung mit natürlicher Sonne, wobei sich die Erfindung darauf bezieht, wenigsten einen Teil des UV-B Lichtanteiles zu filtern. Weiters ist die Vorrichtung auf der sonnenabgewandten Seite weder gegen Wärmeverlust durch Strahlung noch Konvektion geschützt. Die Lichtstrahlen die auf der sonnenzugewandten Seite durch die Folie hindurchgehen werden nur auf der Aufstellfläche und an dem zu Bestrahlenden selbst absorbiert und in Wärme umgewandelt. Da aber die Aufstellfläche nicht isoliert ist, geht diese Wärme gleich wieder verloren und kann die Luft innerhalb der Vorrichtung nicht erwärmen. Speziell in Monaten mit niedrigem Sonnenstand werden Lichtstrahlen ohne in der Vorrichtung absorbiert zu werden an der Rückseite der Vorrichtung durch die Folie wieder austreten. Der Wärmegewinn der Luft im Inneren dieser Vorrichtung fällt daher bei kälteren Außentemperaturen gering aus.

Der Erfindung liegt daher die Aufgabe zu Grunde, einen Bestrahlungsapparat zu konstruieren, der mit natürlichem Sonnenlicht arbeitet und das größtmögliche sowie unverzerrte Sonnenlichtspektrum ins Innere des Apparates läßt, damit die biologischen Prozesse in den Körperzellen durch das Sonnenlicht bestmöglich stimuliert werden können. Außerdem soll der Apparat die Möglichkeit bieten, bei zu niedrigen oder zu hohen Außentemperaturen ebenfalls eine angenehme Teil- oder Ganzkörperbestrahlung zu gewährleisten. Optional soll der Apparat gleichzeitig als Sauna verwendet werden können. Dieser Bestrahlungsapparat soll im Gesundheitsbereich profilaktisch als auch therapeutisch aber ebenso für kosmetische Zwecke wie Hautbräunung einsetzbar sein.

Die Aufgabe wird erfindungsgemäß einerseits dadurch gelöst, daß der Apparat als eine Art Kabine ausgeführt ist, in deren Innenraum sich der zu Bestrahlende begibt. und dessen sonnenlichtdurchlässige Abdeckung das größtmögliche ganzheitliche Spektrum des Sonnenlichtes hindurchläßt, andererseits die restlichen Umschließungsflächen wie Wände, Boden u. Dach derart gestaltet werden, daß Sie den Treibhauseffekt im Inneren des Apparates fördern. Die Temperaturregelung für eine angenehme Teil- oder Ganzkörperbestrahlung erfolgt mit erfindungsgemäßen Konstruktionen und kann aber auch in Kombination mit externen gegen E-Smog abgeschirmten Heiz- bzw. Kühlgeräten durchgeführt werden.

In den Zeichnungen ist der Erfindungsgegenstand beispielsweise dargestellt. Es zeigen:
- Fig. 1: den erfindungsgemäßen Apparat (1) in einer Draufsicht
- Fig. 2: Apparat (1) von der sonnenzugewandten Seite
- Fig. 3: Apparat (1) in einer Seitenansicht
- Fig. 4: Apparat (1) in einer Schrägansicht mit Reflektoren
- Fig. 5: Apparat (1) von der sonnenzugewandten Seite mit Reflektoren
- Fig. 6: Apparat (1) in einer Seitenansicht im Schnitt mit Jalousien
- Fig. 7: den Aufbau der Umschließungsflächen im Schrägschnitt
- Fig. 8: den Aufbau der Umschließungsflächen im Schnitt
- Fig. 9: den Aufbau der Umschließungsflächen Dach, Wand u. Boden mit Ab- Umluftsystem im Schnitt
- Fig. 10: den aufblasbaren Apparat (30) in einer Schrägansicht
- Fig. 11: den aufblasbaren Apparat (30) in einer Seitenansicht im Schnitt mit Sitzfläche

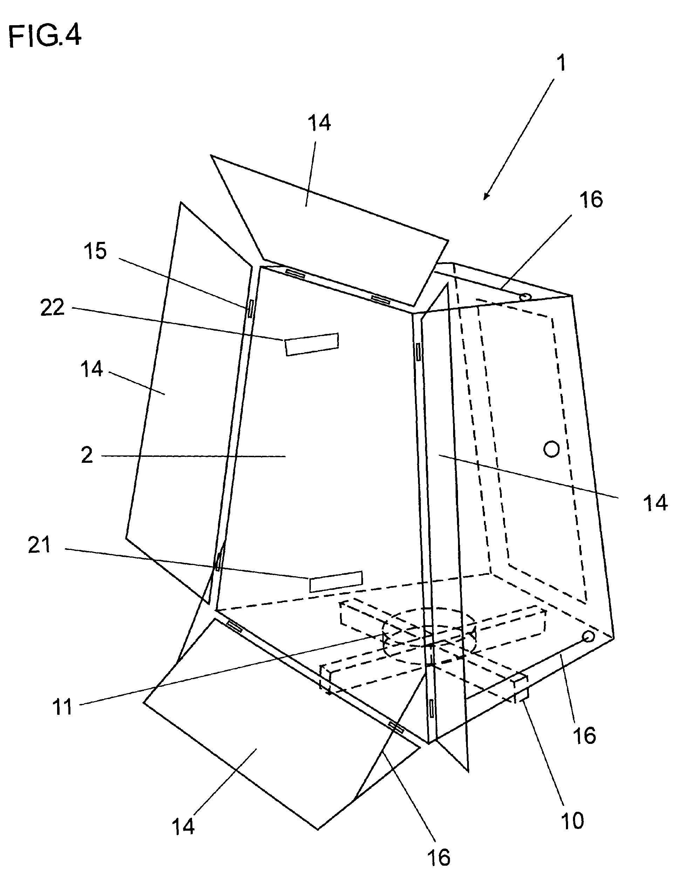

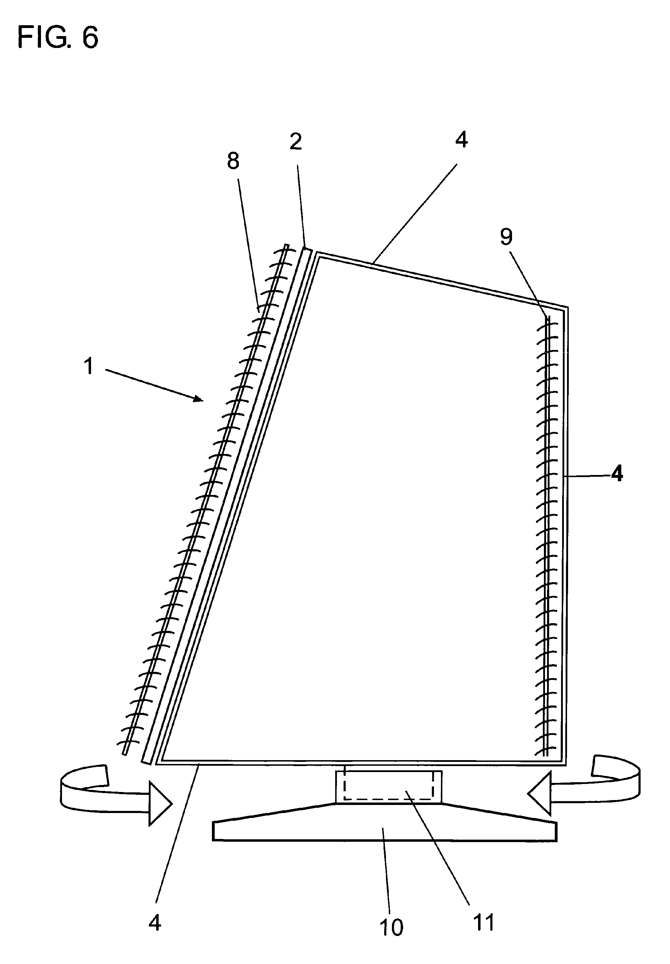

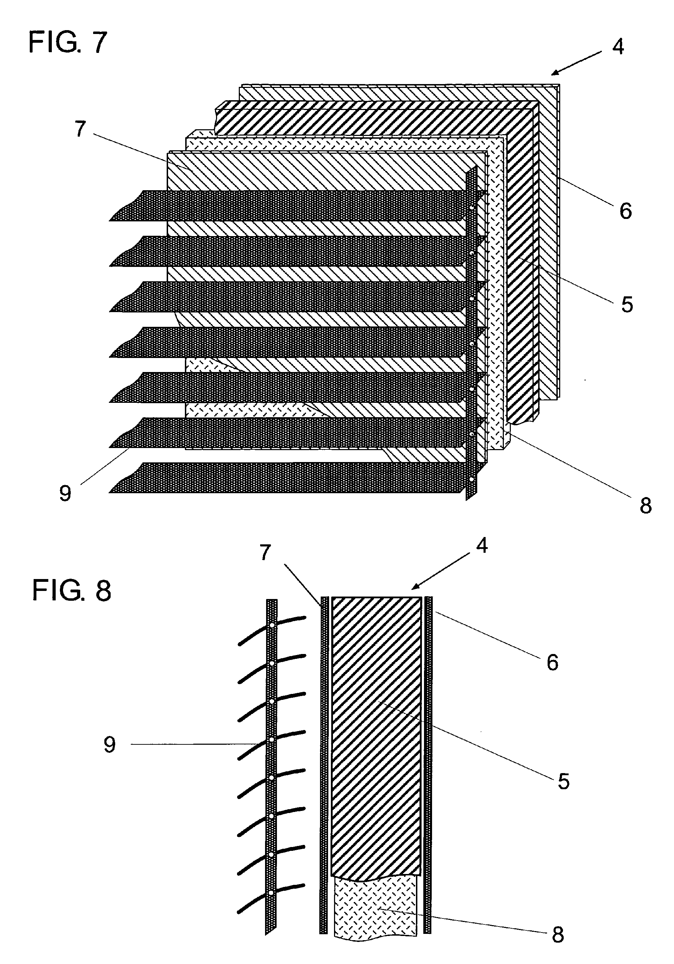

Der Bestrahlungseffekt mit natürlichen Sonnenlicht im ( Fig. 4) Inneren des Apparates (1) wird durch seine transparente Abdeckung (2) oder eine Abdeckung aus Netzen mit entsprechender Maschenweite erzeugt. Die transparente Abdeckung (2) wird vorzugsweise aus Materialien wie Quarzglas, Borosilikatglas, eisenfreies Borosilikatglas oder Polymethylmethacrylat (PMMA) hergestellt, welche das größtmögliche und verzerrungsfreie Spektrum des Sonnenlichts im UV Bereich, dem sichtbaren Bereich, als auch dem IR Bereich hindurchlassen. Aber auch Folien mit diesen Eigenschaften sind einsetzbar. Auch Netze in entsprechender Maschenweite können verwendet werden um zwar Strahlung ins Innere zu lassen, die erwärmte Luft aber nicht mehr entfliehen zu lassen. Die Intensität der Strahlung wird zwar durch das Netz abgeschwächt, und der Treibhauseffekt ist etwas schwächer als bei der transparenten Abdeckung (2), da ja durch das Netz eine gewisse Luftzirkulation möglich ist, vorteilhaft wirkt sich jedoch aus, daß die hindurchgelassene Strahlung in ihrer Frequenz jedoch absolut unverändert bleibt und nur in ihrer Intensität reduziert wird. Der Apparat (1) (Fig 4) wird so positioniert, daß die lichtdurchlässige Abdeckung (2) der Sonne zugewandt ist, während die sonnenabgewandten Umschließungsflächen (4) wie Boden, Dach, Seiten- u. Rückwände wärmegedämmt ausgeführt werden und somit den Treibhauseffekt fördern. Der Treibhauseffekt entsteht dadurch, daß die Sonnenstrahlen die Abdeckung (2) passieren an den Innflächen des Apparates, je nach Ausführung der Innenflächen, mehr oder weniger absorbiert und in Wärme umgewandelt werden. Mindestens eine der Umschließungsflächen (4) oder die Abdeckung (2) ist für einen bequemen Eintritt in den Apparat (1) öffenbar. Die Umschließungsflächen (4) werden vorzugsweise in Elementbauweise gefertigt. Jede Umschließungsfläche(4) (Fig. 7) hat einen umlaufenden Rahmen (5) auf dessen Außenseite eine wetterfeste Verkleidung (6) und auf der Innenseite (7) wahlweise Materialien mit lichtreflektierenden oder lichtabsorbierenden Oberflächen angebracht werden können. Zwischen Innen- (7) und Außenverkleidung (6) befindet sich die Wärmedämmung (8). Das Material der Innenseiten (7) soll in jedem Fall ein geringes Wärmespeichervermögen haben um eine schnelle Aufheizung der Oberfläche und damit eine rasche Wärmeabgabe an den Innenraum des Apparates (1) zu gewährleisten. Hierzu eignen sich entsprechend lackiertes Holz, Metallbleche oder Kunststoffe. Um den Wärmeeintrag durch die eintreffende Strahlung ins Innere des Apparates (1) gegen Überhitzung vor allem in warmen Jahreszeiten bei direkter Strahlung regulieren zu können, können vor der lichtdurchlässigen Abdeckung (2) verstellbare Sonnenschutzbehelfe (8) (Fig. 6) wie Jalousien, Rollos oder Netze befestigt werden. Dadurch verringert sich allerdings auch der Eintrag der Strahlung auf den zu Bestrahlenden was in den Sommermonaten bei direkter Einstrahlung ohne Bewölkung sicherlich von Vorteil ist. Der Wärmeeintrag kann aber auch geregelt werden ohne die Strahlungsintensität auf den zu Bestrahlenden zu verringern, was in Zeiten geringer Strahlungsintensität wichtig ist.

Wie oben beschrieben können die Innen-Oberflächen der Innenwände entweder lichtreflektierend oder -absorbierend ausgeführt werden. Sind die Innenoberflächen absorbierend ausgeführt, werden vor die absorbierenden Innenflächen in ihrer Größe verstellbare Flächen (9) ( Fig. 6,7 u. 8) angebracht, mit deren Größenänderung die Beschattungfläche der Innenwandoberflächen (7) und damit die Absorptionsfläche bzw. Reflexionsfläche eingestellt werden kann. Die verstellbaren Flächen können Jalousien (9) wie dargestellt oder Rollos sein, beide Varianten sind auf einer Seite in lichtabsorbierenden und auf der anderen Seite in reflektierenden Farben ausgeführt . Damit können diese sowohl für Apparate mit absorbierenden als auch reflektierenden Innenwandoberflächen (7) verwendet werden. Sind die Innenwandoberflächen (7) reflektierend gestaltet, werden die verstellbaren Flächen (9) auf der sonnenzugewandten Seite mit Ihrer absorbierenden Fläche angebracht.

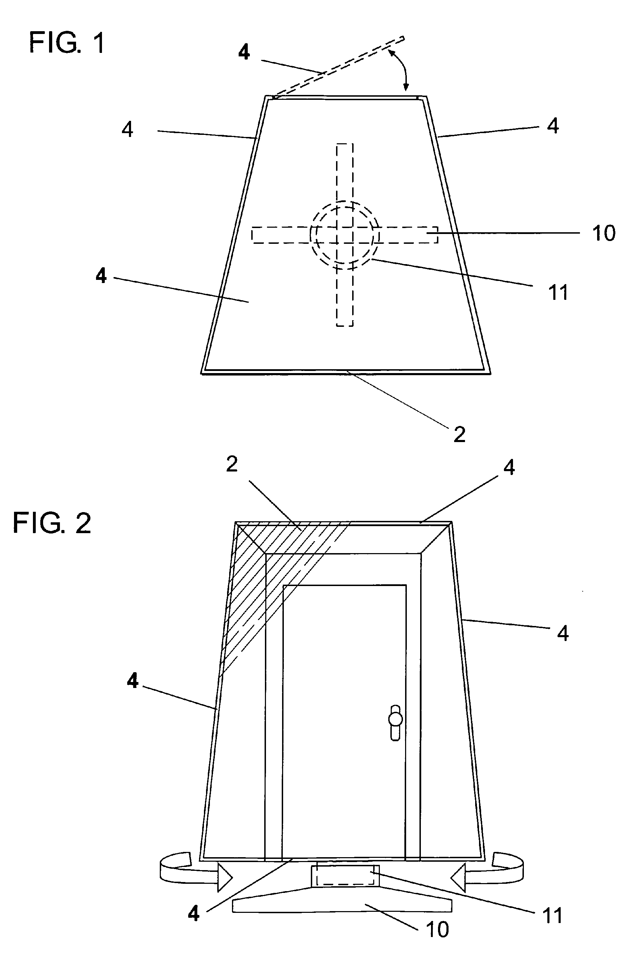

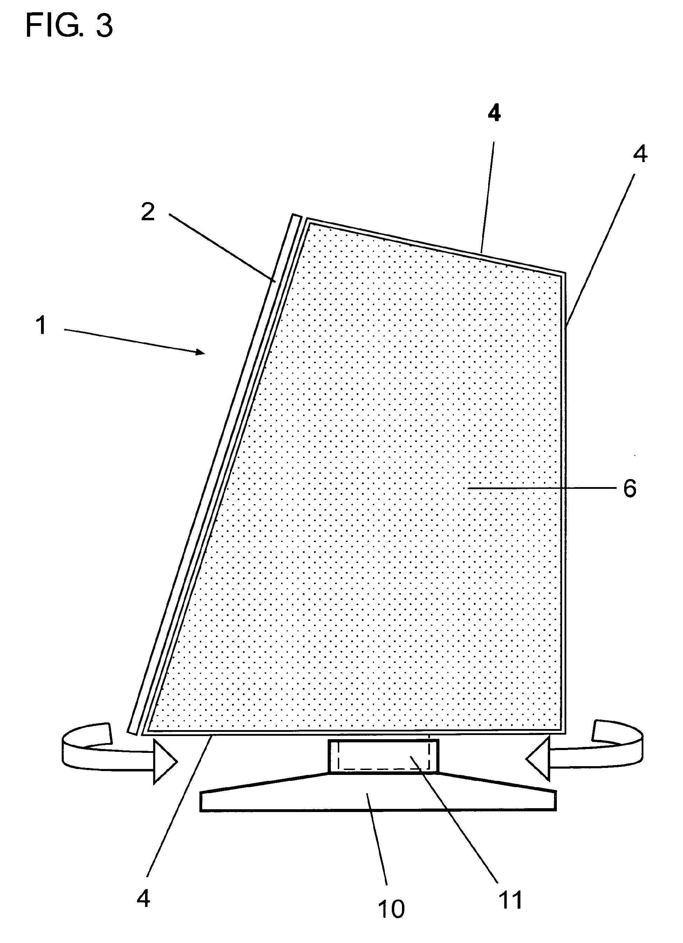

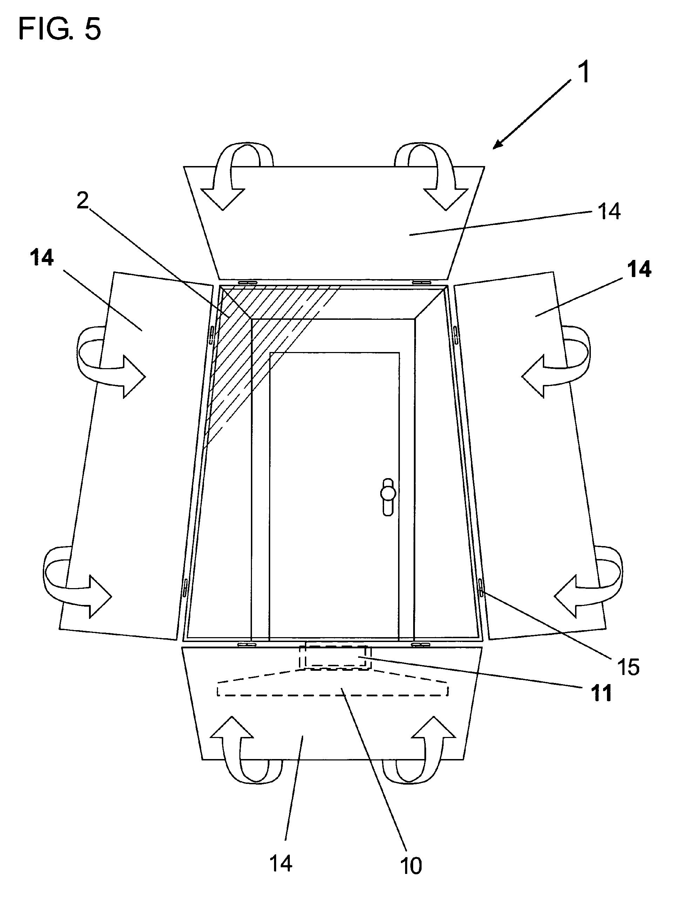

Bedarfsmäßig eingestellt, kann dann die eintreffende Strahlung absorbiert oder nach außen reflektiert werden. Der Apparat (1) wird außerdem gegenüber einem Standfuß (10) (Fig. 3,5 u. 6) durch ein Gelenk oder Lager (11) drehbar oder schwenkbar ausgeführt, damit er der Sonnenbewegung folgend, vom Inneren des Apparates (1) aus, bequem nachgeführt werden kann. Je größer das Verhältnis von Lichteintrittsfläche zu Umschließungsflächen ist, umso wärmer wird es im Innenraum des Bestrahlungsapparates (1). Vorteilhaft ist es auch die Seitenwände der Umschließungsflächen (4) schräg zu gestalten (Fig1), da die Absorptionsfähigkeit der Innenwände größer wird, je näher die Sonnenstrahlen an den Auftreffwinkel von 90 Grad kommen, halbkreisförmige Wandausbildungen sind daher ebenfalls sehr effektiv, dies ist jedoch nicht näher dargestellt. Die warmen Innenflächen des Apparates strahlen behagliche langwellige Wärmestrahlung ab, was besonders bei kühlen Außentemperaturen angenehm ist. Dabei kann es trotz kühler Außentemperaturen und bei auf reflektierend gestellten Flächen (9) passieren, daß es zu warm im Apparat (1) wird, hierfür sind verschließbare Zuluft- (21) (Fig 4) und Abluftklappen (22) in der Umschließungsfläche (4) Wand vorgesehen, die so angeordnet sind, daß die natürliche Thermik der Luft, ein Ausströmen zu warmer Luft ermöglicht oder dieser Effekt noch durch einen gegen E-Smog abgeschirmten Ventilator verstärkt werden kann.

Um einen entsprechenden Energieeintrag bei sehr kalten Außentemperaturen und Bewölkung, also diffusem Strahlungsangebot zu erreichen, werden schwenkbare, vorzugsweise Metall-Reflektoren (14) ( Fig. 4 u. 5) außen am Apparat (1) derart positioniert, daß sie das Sonnenlicht durch die Abdeckung (2) oder das Netz in das Innere des Apparates reflektieren. Die Reflektoren sind mittels Scharnieren (15) befestigt, und können bei Gebrauch und danach variabel mit einem Gestänge (16) fixiert werden um Schnee- oder Windlasten zu widerstehen.

Die zwecks Temperaturregelung im Inneren angebrachten verstellbaren Flächen (9) mit ihrer reflektierenden Seite haben noch eine weitere Funktion und zwar die Reflexion des Lichtes auf die sonnenabgewandte Körperrückseite der zu bestrahlenden Person. Damit wird erreicht, daß der ganze Körper annähernd gleichmäßig bestrahlt wird. Um im Bestrahlungsapparat (1) nicht stehen zu müssen, ist es vorteilhaft die Bespannungen der Sitz- oder Liegebehelfe aus den gleichen lichtdurchlässigen Materialien wie die Abdeckung (2) des Apparates (1) oder aus Netzten auszubilden, was jedoch nicht näher dargestellt wird.

Um eine noch angenehmere Bestrahlung auch bei heißen Außentemperaturen zu ermöglichen, kann die Luft im Inneren des Apparates (1) durch eine Klimaanlage gekühlt werden. Damit die Klimaanlage jedoch kostensparender arbeiten kann, wird man die verstellbaren Flächen (9) auf reflektierend stellen, denn dann wird wenig Strahlung absorbiert und in Wärme umgewandelt.

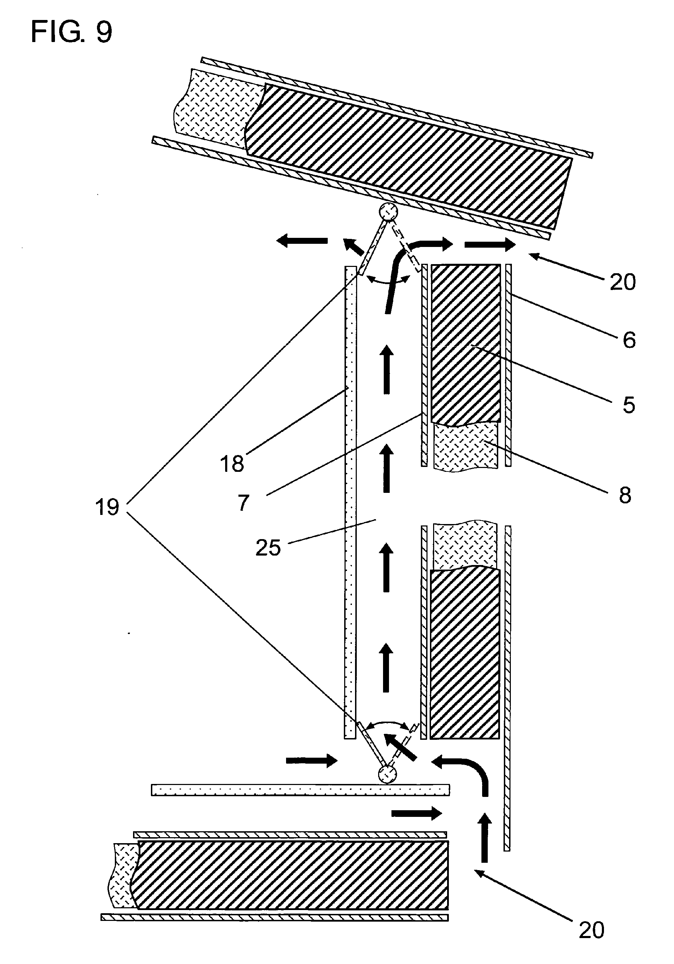

Eine weitere Möglichkeit den Emergieaufwand für die Kühlung zu verringern besteht darin, (Fig. 9) die Innenseiten (7) der Umschließungsflächen der Seiten- u. Rückwände des Apparates aus absorbierenden Materialien wie dunkles Metall, Holz oder Kunststoff, vorzugsweise aber aus Metallblechen mit einer hochselektiven Beschichtung, wie sie bei thermischen Sonnenkollektoren eingesezt werden, auszuführen. Der Emmissionsfaktor des hochselektiven Metallbleches liegt etwa bei 5 %, was dazu führt, daß eine mit einem Luftzwischenraum (25) zum Metallblech (7) auf Abstandhaltern montierte transparente Abdeckung (18) weniger durch langwellige Wärmestrahlung aufgeheizt wird. Diese gibt ihrerseits wieder weniger Wärme an das Innere des Apparates (1) ab. Bei der transparenten Abdeckung (18) ist die Lichtdurchlässigkeit bezüglich Unverzerrtheit des Lichtspektrums bedeutungslos. Eine verstellbare Zuluft- u. Abluftklappe (19) an der Unter- und Oberseite der transparenten Abdeckung (18) sowie Öffnungen (20) in der dahinterliegenden Umschließungsfläche der Seiten- u. Rückwand -ermöglichen es, die sich am Metallblech (7) erwärmte Luft, entweder in den Innenraum des Apparates oder ins Freie abzuleiten. Damit kann bei kühlen Temperaturen Wärme gewonnen werden und bei unerwünschten heißen Temperaturen etwa 90 % der in den Apparat einfallenden Strahlung und in Wärme umgewandelten Energie, nach außen abgeleitet werden.

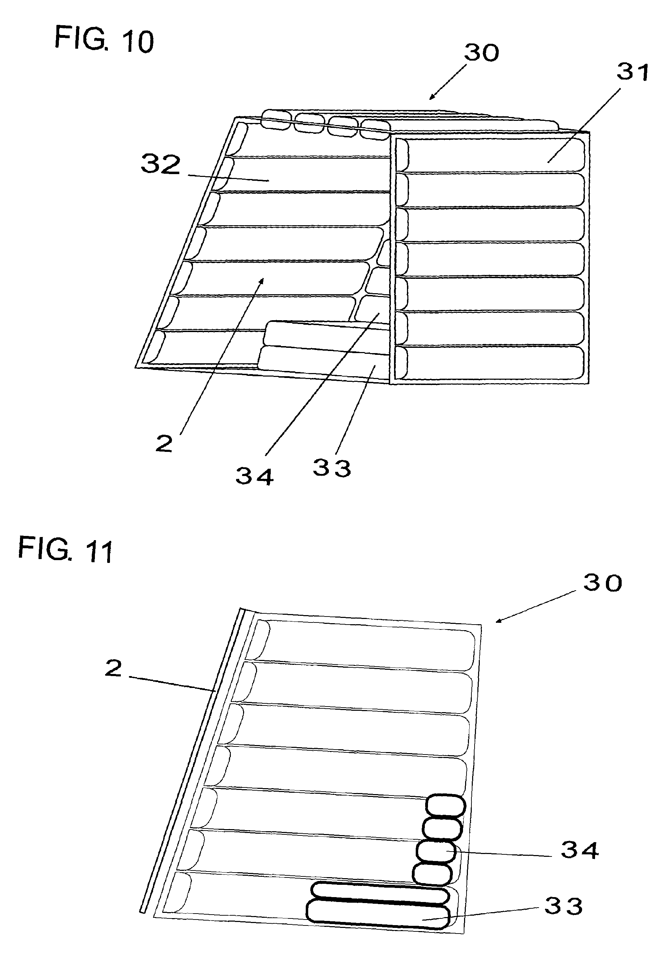

Um eine Ganzkörperbestrahlung zu ermöglichen, wenn das in den Apparat eintreffende Sonnenlicht nicht ausreicht, um die gewünschte Innentemperatur zu erreichen, kann durch eine gegen E-Smog abgeschirmte externe elektrische Heizquelle nachgeholfen werden. Ist diese Heizquelle entsprechend dimensioniert ist der Apparat auch bei zu geringem Strahlungsangebot als Sauna zu verwenden. Der Apparat (1) ausgeführt mit einer wärmegedämten Rahmenkonstruktion ist eine hochwertige Variante, erfindungsgemäß kann aber auch eine leichte aufblasbare und daher kostengünstige Variante des Apparates (30) Fig. 10. gefertigt werden.

Die lichtdurchlässige Abdeckung wird wiederum aus einer das größtmögliche Sonnenlichtspektrum hindurchlassenden Folie oder einem Netz in entsprechender Maschenweite ausgeführt, während die anderen Umschließungsflächen aus einem doppelwandigen und selbsttragendem Luftkammersystem, ähnlich wie bei Luftmatratzen oder Schlauchboten, bestehen. Die Innenwand (32) des Luftkammersystems wird aus dunklem sonnenlichtabsorbierenden Material und die Außenwand (31) vorzugsweise in reflektierender spiegelnder Folie gefertigt. Damit wird die Wärmestrahlung, ausgehend von der inneren dunklen Folie, von der reflektierenden Folie zurückgeworfen, und eine Art Ping-Pong-Effekt erzeugt, der den K-Wert des Luftkammersystems wesentlich verbessert. In Fig. 11 einem Schnitt von der Seite ist die integrierte Sitzfläche (33) und die dazugerhörige Rückenlehne (34) sichtbar. Diese Variante Des Apparates (30) ist speziell für eine Bestrahlung bei kühlen Außentemperaturen einsetzbar. Bei wärmeren Außentemperaturen kann dieser Apparat auch zum Schwitzen bei gleichzeitiger Bestrahlung verwendet werden. Eine weitere Anpassung der Innentemperatur durch externe Geräte oder verstellbare Flächen erfolgt in diesem Falle nicht. Vorteile der aufblasbaren Variante sind, daß dieser Apparat kostengünstig hergestellt werden kann und leicht transportierbar ist, beispielsweise in einem Rucksack zum Ski-laufen oder Winterwandern mitgenommen werden kann.

## Patentansprüche

1. Apparat zur Bestrahlung des menschlichen Körpers, **dadurch gekennzeichnet, daß** die Bestrahlung mit natürlichem Sonnenlicht erfolgt, welches eine Abdeckung passiert, die ein größtmögliches und unverzerrtes Lichtspektrum in das inneres des Apparates läßt.

2. Apparat zur Bestrahlung des menschlichen Körpers, nach Anspruch 1, **dadurch gekennzeichnet, daß** die sonnenabgewandten Umschließungsflächen (4) wärmegedämt ausgeführt sind.

3. Apparat zur Bestrahlung des menschlichen Körpers, nach Anspruch 1 und 2 **dadurch gekennzeichnet, daß** die sonnenabgewandten Umschließungsflächen (4) aus selbsttragenden abgeschloßenen Luftkammern bestehen.

4. Apparat zur Bestrahlung des menschlichen Körpers, nach Anspurch 1,2 und 3 **dadurch gekennzeichnet, daß** die innere Folie (32) der Luftkammern dunkel also absorbierendend und die äußere Folie (31) reflektierend vorzugsweise spiegelnd oder silberfarben ausgeführt ist.

5. Apparat zur Bestrahlung des menschlichen Körpers, nach Anspruch 1 und 2, **dadurch gekennzeichnet, daß** die Innenflächen der Umschließungsflächen (10) aus Materialien mit geringer Wärmekapazität in dunkler Oberfläche ausgeführt sind.

6. Apparat zur Bestrahlung des menschlichen Körpers, nach Anspruch 1,2 und 5, **dadurch gekennzeichnet, daß** die Innenflächen (7) der Umschließungsflächen (4) aus Materialien mit geringer Wärmekapaziät in heller oder reflektierender Oberfläche ausgeführt sind.

7. Apparat zur Bestrahlung des menschlichen Körpers, nach Anspruch 1,2,5, und 6 **dadurch gekennzeichnet, daß** an der Innenseite (7) der Umschließungsflächen (4) größenverstellbare Flächen (9) als Reflektoren dienend angebracht sind.

8. Apparat zur Bestrahlung des menschlichen Körpers, nach Anspruch 1,2,5,6 und 7 **dadurch gekennzeichnet, daß** an den Innenflächen (7) der Umschließungsflächen (4) größenverstellbare Flächen (9) als Absorber dienend angebracht sind.

9. Apparat zur Bestrahlung des menschlichen Körpers, nach Anspruch 1,2,5,6,7 und 8, **dadurch gekennzeichnet, daß** der Bestrahlungsapparat (1) durch ein Gelenk oder Lager (11) gegenüber dem Standfuß (10) um seine horizontale vertikale, oder beide Achsen drehbar ist.

10. Apparat zur Bestrahlung des menschlichen Körpers, nach Anspruch 1,2 5,6,7,8 und 9 **dadurch gekennzeichnet, daß** außen am Apparat (1) zusätzliche verstellbare Reflektoren (14) montiert sind.

11. Apparat zur Bestrahlung des menschlichen Körpers, nach Anspruch 1,2 5,6,7,8,9 und 10 **dadurch gekennzeichnet, daß** die Innenflächen (7) der Umschließungsflächen (4) mit einer auf Abstandhaltern montierten zusätzlichen transparenten Abdeckung (18) versehen sind.

12. Apparat zur Bestrahlung des menschlichen Körpers, nach Anspruch 1,2, 5,6,7,8,9,10 und 11 **dadurch gekennzeichnet, daß** die Luftzwischenräume (25) mittels mechanischen verstellbaren Klappen (19) entweder ins Freie oder in das Innere des Apparates entlüftet werden können.

13. Apparat zur Bestrahlung des menschlichen Körpers, nach Anspruch 1,2, 5,6,7,8,9,10,11,u. 12 **dadurch gekennzeichnet, daß** die Innenfläche (7) aus hochselktivem beschichteten Blech ausgeführt ist.

14. Apparat zur Bestrahlung des menschlichen Körpers, nach Anspruch 1,2, 5,6,7,8,9,10,11,12 und 13 **dadurch gekennzeichnet, daß** die gewünschte Innentemperatur durch externe Wärme- oder Kältegeräte erzeugt wird.

## Geänderte Patentansprüche

### Geänderte Patentansprüche gemäss Regel 137(2) EPÜ.

**1.** Apparat zur Bestrahlung des menschlichen Körpers mit natürlichem Sonnenlicht, bestehend aus wärmegedämmten Umschließungsflächen (4) und einer transparenten Abdeckung (2) **dadurch gekennzeichnet, daß** die Abdeckung (2) aus Glas mit einer Transmission des Spektrums von mindestens 160 - 4000 nm besteht.

**2.** Apparat zur Bestrahlung des menschlichen Körpers mit natürlichem Sonnenlicht, bestehend aus wärmegedämmten Umschließungsflächen (4) und einer transparenten Abdeckung (2) **dadurch gekennzeichnet, daß** die Abdeckung (2) aus Polymethylmethacrylat (PMMA) mit einer Transmission des Spektrums von mindestens 250 - 2300 nm besteht.

**3.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1 und 2, **dadurch gekennzeichnet, daß** die Seitenwände der Umschließungsflächen (4) schräg ausgebildet sind (FIG. 1 u.4) und der Winkel gegenüber der Abdeckung (2) kleiner 90 Grad ist.

**4.** Apparat zur Bestrahlung des menschlichen Körpers mit natürlichem Sonnenlicht, bestehend aus wärmegedämmten Umschließungsflächen (4) und einer transparenten Abdeckung (2) nach Apruch 2 und 3, **dadurch gekennzeichnet, daß** die Umschließungsflächen (4) (FIG. 10) aus doppelwandigen, selbsttragenden, abgeschloßenen, aufblasbaren Luftkammern aus Folie bestehen und die Seitenwände schräg ausgeführt sind.

**5.** Apparat zur Bestrahlung des menschlichen Körpers mit natürlichem Sonnenlicht, bestehend aus wärmegedämmten Umschließungsflächen (4) (FIG. 10) und einer transparenten Abdeckung (2) nach Anspurch 2, 3 und 4, **dadurch gekennzeichnet, daß** die Innenwand (32) der Luftkammern dunkel also absorbierend und die Außenwand (31) reflektierend vorzugsweise spiegelnd oder silberfarben ausgeführt ist.

**6.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1,2 und 3, **dadurch gekennzeichnet, daß** an der Innenseite (7) (FIG. 6) der Umschließungsflächen (4) größenverstellbare und um 180 Grad wendbare Flächen (9) angebracht sind, deren eine Seite mit gut reflektierender Oberfläche (silber oder weiß) und die andere mit dunkler gut absorbierender Oberfläche ausgeführt ist.

**7.** Apparat zur Bestrahlung des menschlichen Körpers mit natürlichem Sonnenlicht, bestehend aus wärmegedämmten Umschließungsflächen (4) und einer transparenten Abdeckung (2) nach Anspurch 1,2,3 und 6, **dadurch gekennzeichnet, daß** der Bestrahlungsapparat (1) durch ein Lager (11) gegenüber dem Standfuß (10) um seine vertikale Achse drehbar gelagert ist, und von diesen gemeinsam die horizontalen und vertikalen Lasten des Bestrahlungsapparates (1) aufgenommen werden.

**8.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1,2,3,6 und 7 **dadurch gekennzeichnet, daß** außen am Apparat (1) (FIG. 4 u.5) vor der transparenten Abdeckung (2) zusätzliche Reflektoren (14) montiert sind, welche in ihrem Winkel verstellbar sind.

**9.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1,2,3,6,7 und 8, **dadurch gekennzeichnet, daß** die Innenflächen (7) der Umschließungsflächen (4) mit einer auf Abstandhaltern montierten, zusätzlichen transparenten Abdeckung (18) (FIG.9) versehen sind.

**10.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1,2,3,6,7,8 und 9, **dadurch gekennzeichnet, daß** der Luftzwischenraum (25) (FIG. 9) mittels mechanischen verstellbaren Klappen (19) entweder ins Freie oder in das Innere des Apparates (1) entlüftet werden können.

**11.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1,2,3,6,7,8,9 und 10, **dadurch gekennzeichnet, daß** die Innenseiten (7) aus hochselektivem beschichteten Blech ausgeführt ist.

**12.** Apparat zur Bestrahlung des menschlichen Körpers mit natürlichem Sonnenlicht, bestehend aus wärmegedämmten Umschließungsflächen (4) und einer transparenten Abdeckung (2) nach Anspruch 1,2,3,6,7,8,9,10 und 11, **dadurch gekennzeichnet, daß** die gewünschte Innentemperatur durch einen elektrischen Infrarotheizstab und eine elektrische Wärmepumpe die heizen und kühlen kann regelbar ist.
